# EUROPEAN PATENT APPLICATION

(11) **EP 2 926 810 A1**
(43) Date of publication of application: **07.10.2015**
(21) Application number: 15161601.8
(22) Date of filing: 30.03.2015
(51) Int. Cl.: A61K 31/19, A61K 47/28, A61K 47/34, A61K 9/08, A61K 9/10

(54) **ORAL LIQUID PHARMACEUTICAL FORMULATIONS OF LOXOPROFEN**

(30) Priority: 31.03.2014 TR 201403702
(71) Applicant: Sanovel Ilac Sanayi ve Ticaret A.S., 34460 Istanbul (TR)
(72) Inventor: Türkyilmaz, Ali, 34460 Istanbul (TR); Erdem, Yelda, 34460 Istanbul (TR); Yag, Göksu, 34460 Istanbul (TR)
(74) Representative: Sevinç, Erkan

(57) **Abstract**

The present invention relates to an oral liquid pharmaceutical formulation comprising loxoprofen or a pharmaceutically acceptable salt thereof and one or more pharmaceutically acceptable excipients.

## Description

### Technical Field of the Invention

The present invention relates to an oral liquid pharmaceutical formulation comprising loxoprofen or a pharmaceutically acceptable salt thereof and one or more pharmaceutically acceptable excipients.

### Background of the Invention

Loxoprofen is a non-steroidal anti-inflammatory drug in the propionic acid derivatives group. It is a prodrug and it is quickly converted to its active trans-alcohol metabolite following oral administration. It is a non-selective cyclooxygenase inhibitor and works by reducing the synthesis of prostaglandins from arachidonic acid. Its chemical name is (RS)-2-{4-[(2-oxocyclopentyl)methyl]phenyl}propanoic acid and its chemical structure is shown in the Formula I.

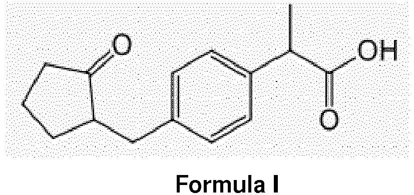

The patent application US4161538 (A) discloses the loxoprofen molecule.

The patent JP2669517 (B2) discloses a solid preparation containing loxoprofen sodium and additives such as microcrystalline cellulose, hydroxypropyl cellulose having low substitution degree, lactose or magnesium stearate, in the form of a tablet, capsule, granule or powder.

The patent EP0947584 (B1) discloses an anti-inflammatory analgesic patch comprising loxoprofen or pharmaceutically acceptable salt thereof, water, crotamiton and a water soluble polymer.

The patent application WO0247661 (A1) discloses pharmaceutical composition for intramuscular injection containing loxoprofen or a pharmaceutically acceptable salt thereof, as an active ingredient.

The patent application JP2010270140 (A) discloses an oral pharmaceutical composition which alleviates gastric mucosa disorders caused by loxoprofen, comprises one or more sugars and sugar alcohols selected from the group consisting of sucrose, maltitol, fructose, xylitol, trehalose, lactose and lactitol; and loxoprofen. A tablet, subtle granule (powder is included), capsule, solution (syrup is included), etc., may be cited as a concrete dosage form of the oral pharmaceutical composition.

In the state of art, there are several patents and applications which disclose many formulations of loxoprofen or a pharmaceutically acceptable salt thereof, in different dosage forms. In the present invention a novel oral solution formulation of loxoprofen or a pharmaceutically acceptable salt thereof is disclosed.

Oral liquid formulations are becoming an increasingly important issue in the area of better patient compliance comparative to the conventional solid dosage forms for oral administration such as capsules and tablets, which are the most commonly used. In particular pediatric and geriatric patients and patients who are mentally retarded or nauseated often experience difficulties in swallowing solid dosage forms. Besides, conventional solid dosage forms are not suitable for bedridden or busy and travelling patients, in case the patient may not have easy access to water. Thus, oral liquid pharmaceutical formulations represent an alternative for such patients and provide for a better patient compliance with recommended pharmaceutical therapies. Unlike the solid dosage forms, oral liquid pharmaceutical forms require no dissolution so its absorption is rapid and unaffected by alterations in gastric pH. Therefore, they deliver high concentration of active pharmaceutical agent to the intestinal epithelium and minimize first-pass metabolism.

On the other side, oral liquid pharmaceutical formulations are not easy to process. A satisfied oral liquid dosage form needs to meet number of requirements such as stability, taste and solubility. Active pharmaceutical agents in liquid forms are more susceptible to chemical and physical instability than the solid state. Also trace amounts of impurities coming from active pharmaceutical agents or excipients and the pH of the solution may cause the degradation of active pharmaceutical agent and this may cause stability problems when the solution is consistently introduced into atmosphere. Moreover, oral liquid pharmaceutical formulations should have pleasant taste for the better patient compliance. Finally, oral liquid pharmaceutical formulations should have desirable solubility, clear appearance without any residue and precipitation. Therefore, stability, taste and solubility are the problems that should be overcome.

### Detailed Description of the Invention

The present invention relates to an oral liquid pharmaceutical formulation comprising loxoprofen or a pharmaceutically acceptable salt thereof and one or more pharmaceutically acceptable excipients.

The objects of this invention are to overcome above mentioned problems related to oral liquid pharmaceutical formulations like obtaining desirable stability, better taste for patients and favorable solubility without any residue or precipitation by using appropriate excipients together and using the suitable excipients in specific ratios.

According to one embodiment, loxoprofen or a pharmaceutically acceptable salt thereof is present in an amount of 0.1 to 20 % and preferably 0.1 to 10 % by weight of total formulation.

The pharmaceutical formulations according to the present invention may also comprise one or more pharmaceutically acceptable excipient(s). Pharmaceutically acceptable excipients comprise, but are not limited to solvents, microbial preservatives, buffering agents, sweeteners, aromatic agents or the mixtures thereof.

In a preferred embodiment of the present invention, said solvents comprise, but are not limited to propylene glycol, water, maltitol solution, glycerine, alcohols, polyethylene glycol, ethanol, isopropyl alcohol or the mixtures thereof, preferably, they are propylene glycol, water and maltitol solution.

In present invention, the oral liquid pharmaceutical formulation comprises propylene glycol and water as solvents to achieve a favorable solubility, desirable solution for delivering the desired dose for a spoonful and better stability. Propylene glycol has become widely used as a solvent in a variety of parenteral and nonparenteral pharmaceutical formulations. It is a better general solvent and dissolves a wide variety of materials, such ascorticosteroids, phenols, sulfa drugs, barbiturates, vitamins (A and D), most alkaloids, and many local anesthetics. Also, water is widely used as a solvent in the processing, formulation and manufacture of pharmaceutical products and it is chemically stable in all physical states. Moreover, maltitol solution is used in this invention and it is noncrystallizing and therefore prevents 'cap-locking' in oral liquid pharmaceutical formulations.

Therefore, in this invention, it has surprisingly been found that the use of propylene glycol, water and maltitol solution together create a synergistic effect over solubility and provides a favorable solution without undesired colloidal precipitation and crystallization. Furthermore, interms of stability, propylene glycol is stable in a well-closed container at cool temperatures, but at high temperatures, in the open, it tends to oxidize. In this invention propylene glycol is used with water because it is chemically stable when mixed with water. Therefore, better stability is achieved with using propylene glycol with water. Also using maltitol solution with paraben mixtures, methyl paraben and propyl paraben is used here, contributes to obtaining desired stability.

According to one embodiment, propylene glycol is present in an amount of 1 to 20 % and preferably 2 to 15 % by weight of total formulation.

According to one embodiment, maltitol solution is present in an amount of 60 to 96 % and preferably 85 to 95 % by weight of total formulation.

In a preferred embodiment of the present invention, said microbial preservatives comprise, but are not limited to methyl paraben, propyl paraben, benzoic acid, boric acid, sorbic acid or their salts thereof, benzyl alcohol, benzalkonium chloride, parahydroxybenzoic acids and their alkyl esters, or the mixtures thereof, preferably, they are methyl paraben and propyl paraben.

According to one embodiment, methyl paraben is present in an amount of 0.01 to 8 % and preferably 0.01 to 5 % by weight of total formulation and propyl paraben is present in an amount of 0.01 to 8 % and preferably 0.01 to 5 % by weight of total formulation.

In a preferred embodiment of the present invention, said buffering agents comprise, but are not limited to citric acid, trisodium citrate dihydrate, ascorbic acid, acetic acid, tartaric acid, citric acid monohydrate, sodium citrate, potassium citrate, sodium phosphate, tricalcium phosphate, calcium carbonate, sodium bicarbonate, calcium phosphate, carbonated calcium phosphate, magnesium hydroxide, hydrochloric acid, sodium hydroxide or the mixtures thereof, preferably, they are citric acid and trisodium citrate dihydrate.

According to one embodiment, citric acid is present in an amount of 0.01 to 3 % and preferably 0.01 to 1 % by weight of total formulation and trisodium citrate dihydrate is present in an amount of 0.1 to 5 % and preferably 0.1 to 1 % by weight of total formulation.

In a preferred embodiment of the present invention, said sweeteners comprise, but are not limited to sucralose, ammonium glycyrrhizinate, maltitol solution, shaccarin sodium, sucrose, aspartame, glucose, lactose, fructose, other sugars, sorbitol, xylitol, erythritol, other sugar alcohols or the mixtures thereof, preferably, they are sucralose, ammonium glycyrrhizinate and maltitol solution.

According to this embodiment, sucralose is present in an amount of 0.01 to 5 %, preferably 0.01 to 2 % by weight of total formulation, ammonium glycyrrhizinate is present in an amount of 0.1 to 5 %, preferably 0.1 to 3 % by weight of total formulation.

The oral liquid pharmaceutical formulations of this invention comprise sucralose as a sweetener to improve patient compliance. In prior art, it is know that aspartame is used mostly as sweetner but contradictory to the prior art we have found that the effect of sucralose as a sweetner in this formulation, not only helped to improve its taste but also increased the efficacy and the conveniency of the formulation because of its positive effects over the glycemic index. There are lots of disadvantages about aspartame and it has a limited usage if you have to use it every day and also there are several incompatibilities reported in literature and safety problems *(*Handbook of Pharmaceutical Excipients, Reymond C Rowe, Paul J Sheskey, Marian E Quinn, sixth edition, pages 48-50*)*. Thus, sucralose has an important role in this aspect and even if it is used in low amounts, it is very important issue in oral liquid pharmaceutical formulations in terms of taste improvement.

Additionally, the oral liquid pharmaceutical formulations of this invention comprise also ammonium glycyrrhizinate as a sweetener in order to obtain the most desirable taste for patients. Ammonium glycyrrhizinate is a product derived from licorice root and it has an extremely sweet taste. It provides a consistent taste in the mouth and even very small amount is sufficient to achieve the desired taste. Ammonium glycyrrhizinate can be used in combination with other sweeteners since it creates a synergistic effect with both natural and synthetic sweeteners. In this invention, it is used with sucralose and maltitol solution, and it has been surprisingly found that the combination of ammonium glycyrrhizinate, sucralose and maltitol solution provides the most pleasant taste for patients.

In a preferred embodiment of the present invention, said aromatic agents comprise, but are not limited to fruit aromas such as orange, banana, strawberry, cherry, wild cherry, lemon, other aromas such as cardamom, anis, mint, menthol, vanillin, tutti frutti or the mixtures thereof.

According to this embodiment, aroma is present in an amount of 0.1 to 10 %, preferably 0.1 to 5 % by weight of total formulation.

The formulation according to the present invention may be in the form of an oral solution.

In this present invention, the formulation has been designed comprising the following:
a) 0.1 to 10 % by weight of loxoprofen
b) 0.01 to 5 % by weight of methyl paraben
c) 0.01 to 5% by weight of propyl paraben
d) 2 to 15 % by weight of propylene glycol
e) 0.1 to 1 % by weight of trisodium citrate dihydrate
f) 0.01 to 1 % by weight of citric acid
g) 85 to 95 % by weight of maltitol solution
h) 0.01 to 2 % by weight of sucralose
i) 0.1 to 3 % by weight of ammonium glycyrrhizinate
j) 0.1 to 5 % by weight of aroma
k) q.s. amount up to 200 ml of pure water

### Example 1 - Oral liquid pharmaceutical formulation of loxoprofen

| **Ingredients** | **Amount** (**mg**)/**200ml** |
|---|---|
| Loxoprofen sodium hydrate | 1200 |
| Methyl paraben | 400 |
| Propyl paraben | 56 |
| Propylene glycol | 5200 |
| Trisodium citrate dihydrate | 480 |
| Citric acid | 74 |
| Maltitol solution | 130000 |
| Sucralose | 20 |
| Ammonium glycyrrhizinate | 200 |
| Aroma | 50 |
| Pure water | q.s. (200ml) |
| **Total weight** | **137680** |

| | |
|---|---|
| *q.s.: quantum sufficient* | |

The pharmaceutical formulation mentioned above is prepared as following:
a) Water is added to a container and heated up to 80 ºC and ammonium glycyrrhizinate is added to the heated water and mixed until it dissolves. The obtained solution is cooled at room temperature. Trisodium citrate dihydrate and citric acid (anhydrous) are added to the mixture and dissolved. Loxoprofen sodium hydrate and sucralose is added to the homogeneous mixture and dissolved by mixing. Then, maltitol solution is added to the mixture and mixed for at least 20 minutes.
b) Propylene glycol, methyl paraben, and propyl paraben are added to another container and dissolved by mixing for at least 2 hours. The mixture is added to the mixture (a) and mixed for 30 minutes and then aroma is added and mixed. The volume of prepared mixture is completed with water. Then, a sample is prepared for pH measurement and if necessary, the pH value is adjusted to 6.0 ± 0.05 with diluted sodium hydroxide solution (4 g/100 ml). The final mixture is filtered by filtration. After filtration process, if the result of the analysis is appropriate the mixture is filled to the bottles by filling and capping process.

## Claims

1. An oral liquid pharmaceutical formulation comprising loxoprofen or a pharmaceutically acceptable salt thereof and one or more pharmaceutically acceptable excipients.

2. The oral liquid pharmaceutical formulations according to claim 1, wherein loxoprofen or a pharmaceutically acceptable salt thereof is present in an amount of 0.1 to 20 % and preferably 0.1 to 10 % by weight of total formulation.

3. The oral liquid pharmaceutical formulations according to claim 1 or 2, wherein one or more pharmaceutically acceptable excipient is selected from the group comprising solvents, microbial preservatives, buffering agents, sweeteners, aromatic agents or the mixtures thereof.

4. The oral liquid pharmaceutical formulations according to any of the preceding claims, wherein the solvent is selected from the group comprising propylene glycol, water, maltitol solution, glycerine, alcohols, polyethylene glycol, ethanol, isopropyl alcohol or the mixtures thereof, preferably, they are propylene glycol, water and maltitol solution.

5. The oral liquid pharmaceutical formulations according to any of the preceding claims, wherein propylene glycol is present in an amount of 1 to 20 % and preferably 2 to 15 % by weight of total formulation.

6. The oral liquid pharmaceutical formulations according to any of the preceding claims, wherein maltitol solution is present in an amount of 60 to 96 % and preferably 85 to 95 % by weight of total formulation.

7. The oral liquid pharmaceutical formulations according to any of the preceding claims, wherein the microbial preservative is selected from the group comprising methyl paraben, propyl paraben, benzoic acid, boric acid, sorbic acid or their salts thereof, benzyl alcohol, benzalkonium chloride, parahydroxybenzoic acids and their alkyl esters, or the mixtures thereof, preferably, they are methyl paraben and propyl paraben.

8. The oral liquid pharmaceutical formulations according to any of the preceding claims, wherein methyl paraben is present in an amount of 0.01 to 8 % and preferably 0.01 to 5 % by weight of total formulation and propyl paraben is present in an amount of 0.01 to 8 % and preferably 0.01 to 5 % by weight of total formulation.

9. The oral liquid pharmaceutical formulations according to any of the preceding claims, wherein the buffering agent is selected from the group comprising citric acid, trisodium citrate dihydrate, ascorbic acid, acetic acid, tartaric acid, citric acid monohydrate, sodium citrate, potassium citrate, sodium phosphate, tricalcium phosphate, calcium carbonate, sodium bicarbonate, calcium phosphate, carbonated calcium phosphate, magnesium hydroxide, hydrochloric acid, sodium hydroxide or the mixtures thereof, preferably, they are citric acid and trisodium citrate dihydrate.

10. The oral liquid pharmaceutical formulations according to any of the preceding claims, wherein citric acid is present in an amount of 0.01 to 3 % and preferably 0.01 to 1 % by weight of total formulation and trisodium citrate dihydrate is present in an amount of 0.1 to 5 % and preferably 0.1 to 1 % by weight of total formulation.

11. The oral liquid pharmaceutical formulations according to any of the preceding claims, wherein the sweetener is selected from the group comprising sucralose, ammonium glycyrrhizinate, maltitol solution, shaccarin sodium, sucrose, aspartame, glucose, lactose, fructose, other sugars, sorbitol, xylitol, erythritol, other sugar alcohols or the mixtures thereof, preferably, they are sucralose and ammonium glycyrrhizinate.

12. The oral liquid pharmaceutical formulations according to any of the preceding claims, wherein sucralose is present in an amount of 0.01 to 5 %, preferably 0.01 to 2 % by weight of total formulation, ammonium glycyrrhizinate is present in an amount of 0.1 to 5 %, preferably 0.1 to 3 % by weight of total formulation.

13. The oral liquid pharmaceutical formulations according to any of the preceding claims, wherein the aromatic agent is selected from the group comprising fruit aromas such as orange, banana, strawberry, cherry, wild cherry, lemon, other aromas such as cardamom, anis, mint, menthol, vanillin, tutti frutti or the mixtures thereof.

14. The oral liquid pharmaceutical formulations according to any of the preceding claims, wherein aroma is present in an amount of 0.1 to 10 %, preferably 0.1 to 5 % by weight of total formulation.

15. The oral liquid pharmaceutical formulation according to any preceding claim comprising;
a) 0.1 to 10 % by weight of loxoprofen
b) 0.01 to 5 % by weight of methyl paraben
c) 0.01 to 5% by weight of propyl paraben
d) 2 to 15 % by weight of propylene glycol
e) 0.1 to 1 % by weight of trisodium citrate dihydrate
f) 0.01 to 1 % by weight of citric acid
g) 85 to 95 % by weight of maltitol solution
h) 0.01 to 2 % by weight of sucralose
i) 0.1 to 3 % by weight of ammonium glycyrrhizinate
j) 0.1 to 5 % by weight of aroma
k) q.s. amount up to 200 ml of pure water
